# EUROPEAN PATENT APPLICATION

(11) **EP 4 684 814 A1**
(43) Date of publication of application: **28.01.2026**
(21) Application number: 24191158.5
(22) Date of filing: 26.07.2024
(51) Int. Cl.: A61M 5/142, A61M 5/145, A61M 5/168, A61M 39/22, F04B 1/0452, F04B 7/00, A61J 1/14, A61J 1/20

(54) **VALVE FOR A MEDICAL FLUID DOSING DEVICE**

(71) Applicant: Medizinische Universität Graz, 8010 Graz (AT)
(72) Inventor: REGITTNIG, Werner, 8010 Graz (AT)
(74) Representative: Gramm, Lins & Partner Patent- und Rechtsanwälte PartGmbB

(57) **Abstract**

The invention relates to a valve for a medical fluid dosing device with a cylinder (1) and a piston (2), which is mounted in the cylinder (1) so as to be longitudinally displaceable and forming a variable-volume working chamber (6) with the cylinder (1), with an actuator (33), which displaces the piston (2) along the longitudinal extension of the cylinder (1) and draws the fluid from a fluid reservoir (9), which is fluidically coupled to the cylinder (1), and pumps it through the valve (13) to a discharge point (23) when the movement is reversed, wherein the valve (13) has a hollow needle (58) with an inlet opening (580) for the fluid, a closed tip (60) opposite the inlet opening (580) and a radial side opening (59), the hollow needle (58) is mounted longitudinally displaceably in a bushing (53) between a closed position and an open position, wherein in the open position the side opening (59) and the closed tip (60) project into a lumen or chamber (57), from which an outlet channel (68) leads, whereby in the closed position the side opening (59) is arranged between two seals (50, 51) in a spacer sleeve (69).

## Description

The invention relates to a valve for a medical fluid dosing device, wherein the fluid dosing device has a cylinder and a piston which is mounted in the cylinder so as to be longitudinally displaceable and forms a variable-volume working chamber with the cylinder, as well as an actuator which displaces the piston along the longitudinal extent of the cylinder, and also a fluid reservoir which is fluidically coupled to the cylinder, so that upon reversal of the movement of the piston, the fluid is pumped from the fluid reservoir through the valve to a discharge point, wherein the valve has a hollow needle with an inlet opening, a closed tip and a radial side opening and is mounted so as to be longitudinally displaceable in a bushing between a closed position and an open position; the invention also relates to such a fluid dosing device. Such a fluid dosing device is designed in particular as a dosing device for medical active substances, in particular insulin.

To treat diabetes, it may be necessary to administer insulin to the patient. Patients are increasingly using an insulin pump for this purpose. The pumps used can be divided into two groups, a so-called tethered pump and a patch pump. Tethered pumps use a thin, flexible tube of varying lengths to connect the cannula inserted under the skin to the pump's insulin reservoir. This allows the patient to wear the pump in comfortable places and infuse insulin in more distant locations, such as the thighs and upper arms.

In contrast to the tetheredp ump, the patch pump integrates the insulin reservoir, the connecting tube, the pump mechanism and the upper part of the subcutaneous cannula in a small housing. This allows the user to place the pump directly on the skin over the infusion site. Patch pumps are generally smaller than tethered pumps but require an additional remote control to set and program the amount of insulin to be delivered.

The standard concentration of insulin preparations is 100 insulin units per millilitre; the insulin pumps on the market are adapted to this concentration. Recently, insulin preparations have been developed that have a much higher concentration, for example 500 insulin units per millilitre; some of these preparations have similarly fast-acting properties as conventional insulin preparations. Thus, it would be desirable to administer these new, fast-acting, higher-concentration insulin preparations via insulin pumps as well. However, safe use of these new insulin preparations requires a very high degree of accuracy in their administration. Contemporary insulin pumps may not meet these high requirements.

A micropipette is known from US 3,828,987 with which a preselected quantity of a fluid can be dispensed. The micropipette comprises a capillary in which a plunger is arranged in order to aspirate or dispense the fluid.

US 4,931,050 relates to an implantable infusion pump that can deliver infusion fluids at different rates and at a constant pressure. A needle is longitudinally displaceable within a capillary. The needle can be displaced over a variable length within the capillary.

US 5,622,482 relates to a pump having a cylinder with an inlet and an outlet and a piston which is slidably mounted within the cylinder between a first position and a second position in order to move fluid from the inlet to the outlet. The piston is held in a first position by biasing means. A shape memory alloy element is used as a drive to move the piston from the first position to the second position, overcoming the biasing force.

US 8,915,893 B2 relates to implantable devices and in particular implantable pumps of a reduced size that are programmable to deliver a liquid drug or other fluid at different rates to a selected site on the human body. A first pressure sensor, a second pressure sensor, an actuating mechanism, a motor, an eccentric shaft and a flexible diaphragm are arranged in a hermetically sealed housing with a sealed interior. A valve unit is in contact with the flexible membrane and is moved by a bending of the membrane due to the movement of the shaft.

US 7,959,606 B2 relates to a method for pumping a predetermined dose of insulin with an insulin pump which has a plunger to perform a reciprocating movement in a cylinder. The cylinder is connected to a supply line and a discharge line, in each of which a non-return valve is arranged. The plunger is driven by a shape memory alloy.

The valves in the devices regulate and control the flow of fluids, for example by partially blocking, fully opening or fully closing passages. When using pumping devices in which a piston or a plunger draws the fluid from a reservoir into a cylinder and, after a reversal of movement, pushes it out of the cylinder to a patient interface, e.g. via a cannula inserted into the subcutaneous fatty tissue of a patient, the cylinder and the entire system must be sealed off from the tissue during the suction movement to prevent fluid from flowing from the tissue into the cylinder. Similarly, during the ejection movement, it is necessary to seal the reservoir against the cylinder to prevent the fluid from being pushed back into the reservoir when it is to be delivered to the patient. This requires appropriate valves that allow the fluid to flow as freely as possible in the desired direction, but reliably prevent it from flowing back against the desired direction of flow.

When used in portable devices for the delivery of liquid drugs, valves should be as small as possible, have low energy consumption for opening and closing them, and have a very good seal in the closed state with a high threshold pressure before a breakthrough occurs. The sealing properties of the valves are particularly important for devices delivering drugs like insulin.

US 5 632 606 A discloses a valve system in which three seals are arranged at equal intervals in an elongated cavity of a valve housing, so that a total of four fluid chambers are formed in the valve housing. A needle, in which a connecting channel is formed, is guided through the seals, so that, depending on the positioning of the needle, fluid can be passed from one fluid chamber into the other fluid chamber on the other side of the seal. The seals are in close frictional contact with the outer circumference of the needle, and thereby preventing fluid leakage between the seals and the needle

US 2020/0025310 A1 discloses a valve system with a valve body that has an inlet channel and an outlet channel. The inlet channel and the outlet channel are connected to a valve needle which is displaceably guided in the valve body. In a first position, a lateral opening in the valve needle is brought into overlap with the inlet channel so that a fluid can flow through the inlet channel into the hollow needle. If the hollow needle is moved in the direction of the inlet channel, the fluid can be transported from the hollow needle into the outlet channel. In an intermediate position, the inlet channel and the outlet channel are closed by the hollow needle.

US 10 583 244 B1 relates to a valve arrangement with two chambers that are separated from each other by a seal. From the outside, a hollow needle passes through a further seal into a first chamber and, if necessary, through the one seal between the two chambers into the second chamber and connects the two chambers to a reservoir. The two seals are configured in such a way that they prevent liquid from escaping when they are pierced by the hollow needle. After the hollow needle is pulled out, the two seals re-seal themselves due to the elasticity of the seals.

US 2023/0094194 A1 relates to a valve comprising a pump chamber having one or more openings, one or more septa covering the one or more openings, a first needle and a second needle. In a first state, a lumen of the first needle is in fluid communication with the pump chamber and a lumen of the second needle is covered by one of the one or more septa. In a second state, the lumen of the second needle is in fluid communication with the pump chamber and the lumen of this first needle is covered by one of the one or more septa.

WO 2021/050494 A1 is related to a drug delivery device comprising a housing defining a shell and an inner volume. A container is at least partially disposed within the housing, the container having an inner volume to contain a medicament. An activation mechanism is at least partially disposed within the housing, the activation mechanism adapted to exert a force to urge the medicament out the container. A needle insertion mechanism is at least partially disposed within the housing, the needle insertion mechanism adapted to insert a needle and/or a cannula to deliver the medicament. A fluid flow connection is coupled with the container and the needle insertion mechanism, the fluid flow connection is adapted to allow the medicament to flow from the container to the needle insertion mechanism. A valve is in fluid communication with the fluid flow connection and the needle insertion mechanism, the valve being movable between at least a first position and a second position, wherein upon the needle insertion mechanism inserting the needle and/or the cannula, the valve is configured to remain in the first position whereby fluid flow is restricted, and at a later time, the valve is urged to the second position whereby fluid may flow through the needle or cannula. The needle may have a closed tip with an opening that can be aligned with a void between a sealing sleeve an the cannula.

The task of the present invention is to improve existing valve arrangements and fluid dosing devices in such a way that they meet the high requirements for leakproofness while requiring as little space and energy as possible.

This task is solved by a valve for a medical fluid dosing device with the features of the main claim and a medical fluid dosing device with the features of the second independent claim. Advantageous embodiments and further embodiments of the invention are disclosed in the subclaims, the description and the figures.

The valve for a medical fluid dosing device, which is provided with a cylinder and a piston which is longitudinally displaceable mounted in the cylinder and which forms a variable-volume working chamber with the cylinder, and with an actuator which displaces the piston along the longitudinal extent of the cylinder and draws the fluid from a fluid reservoir which is fluidically coupled to the cylinder and pumps it to a discharge point when the movement is reversed, provides that a hollow needle is formed with an inlet opening, a closed tip and a radial side opening and that the hollow needle is longitudinally displaceably mounted in a bushing between a closed position and an open position, and in that in the open position the side opening and the closed tip protrude into a lumen or chamber from which an outlet channel leads. The hollow needle has an inlet opening, particularly at its rear end, through which the fluid is fed from the cylinder through the piston, possibly through a feed line. The pressurized fluid is conveyed through the inlet opening and the hollow needle to the radial side opening, whereby the radial side opening is located in front of the tip in the direction of flow from the inlet opening to the tip. In particular, a cavity is formed between the front end of the side opening and the closed tip of the hollow needle. When the hollow needle is in the open position, both the closed tip and the radial side opening protrude into the chamber or lumen that extends around the hollow needle within the bushing. The chamber or lumen can be a cross-sectional enlargement of an insertion channel of the hollow needle, which is formed or arranged in the bushing. The insertion channel is sealed with respect to the hollow needle so that, in the open state or position, fluid is transported and forced by the piston from the cylinder through a conduit and the inlet port and from there through the hollow needle into the chamber or lumen. The excess pressure generated in the chamber or lumen causes the fluid to be pressed out of the outlet channel of the chamber or lumen and thus out of the bushing. In the outlet channel, either a cannula leads directly to the place where the fluid is to be administered or to a line and a channel that ends at such a cannula or delivery point.

In the closed position, the radial side opening does not protrude into the chamber or lumen, but is in the insertion channel to the bushing, possibly in a sealed state, so that no fluid can flow from the lumen into the radial side opening. The lumen is sealed by the closed tip of the hollow needle and the seal surrounding the hollow needle and the valve prevents the fluid from flowing back.

In a closed position, the hollow needle is positioned such that the side opening is arranged between two seals in a spacer sleeve, the spacer sleeve spacing two seals apart and forming an intermediate volume upstream of the lumen that is in communication with the outlet channel. Such a design ensures, on the one hand, that no fluid is transported from the side opening through the outlet channel in the closed position and, on the other hand, that a double seal can be made against fluid escaping in the opposite direction through the inlet opening of the hollow needle. Both seals can be arranged in the seal holder and coupled to the bushing via the seal holder. The seal or seals can be designed so that they are not wider than the length of the side opening, which can then reduce the contact pressure and thus the friction.

In one embodiment, the seal or septum is arranged in a seal holder or septum holder that is fixed to the bushing. The seal holder at least partially surrounds the bushing and advantageously has a passage channel for the hollow needle. The seal holder can be positively fixed to the bushing, for example screwed on or secured by a screw connection. This makes it possible to detach the seal holder from the bushing and replace a seal if necessary. Alternatively, the seal holder is permanently attached to the bushing, whereby the seal holder preferably enables attachment to a valve housing. In one embodiment, a corresponding form-locking element is formed or arranged on the seal holder, for example a thread, a spring arrangement, a latching recess or the like, in order to secure the seal holder to the housing. This makes it possible to first insert the bushing into the valve housing, then to provide a seal and to fasten and mount this seal together with the bushing in the valve housing in such a way that the volume or lumen is sealed on the one hand and the bushing is mechanically fixed within the valve housing on the other.

In one embodiment of the valve, the hollow needle is guided into the bushing by a capillary, whereby the capillary, for example a metal tube or a glass tube, is fixed and secured in the bushing or in the seal holder. The capillary guides the hollow needle in a movable manner and simultaneously supports it so that longitudinal displacement along the length of the capillary is possible. There is a transition fit between the capillary and the hollow needle, in which the clearance between them is chosen to be so small that the hollow needle can be moved back and forth in the capillary without great resistance and at the same time the flow of the fluid between the hollow needle and the capillary is made more difficult and is only possible with an increased fluid pressure. Such transition fit provides a high level of tightness and can be used alone or in conjunction with an additional seal or an additional seal.

The capillary can have an end seal, which is located, for example, at the mounting point of the capillary inside the bushing or the seal holder. Alternatively or additionally, the seal is arranged at the front end of the capillary in the direction of the inlet opening of the hollow needle, so that an effective seal is provided at the entry point of the hollow needle into the capillary.

In one embodiment, the bushing is inserted into the valve housing and positively secured therein; it can be secured, for example, by the seal holder. In principle, it is also possible to secure the bushing separately within the valve.

In one embodiment of the invention, the bushing and the outlet channel are formed in one piece, with the outlet channel extending advantageously in the direction of insertion and reciprocal direction of movement of the hollow needle. The fluid guided through the hollow needle thus exits radially laterally from the hollow needle through the side opening, flows into the lumen and out of the lumen through the outlet channel.

In one embodiment, the valve is coupled to an actuator which is designed, for example, as a spring or has an energy storage device or is alternatively or additionally coupled to a shape memory element. The actuator moves the valve from the open position to the closed position and back, whereby a preferred position is the closed position into which the hollow needle is moved via a spring when there is no pumping movement by the piston. This closes the entire system and prevents fluid from being transported from the fluid reservoir through the outlet channel to an outlet port and discharge point.

In particular, if the spacer sleeve is arranged between two seals, it is possible for an inlet line to open into the spacer sleeve and be in fluidic connection with the side opening in the closed position. This makes it possible to design the valve as a multiport valve that can draw fluid from the fluid reservoir through the spacer sleeve and the side opening during a suction movement in the closed position. If the hollow needle is then moved from the closed position to the open position, the rear, closed part of the hollow needle bridges the area of the spacer sleeve, while the side opening of the hollow needle protrudes into the lumen and fluid can be transported through the lumen to the dispensing point by a pumping movement of the piston in the cylinder. At least one of the seals should be at least as wide as the length of the side opening so that no fluid can flow between the inlet line and the lumen when the side opening is moving through this seal.

The seal according to any embodiment is made of an elastomer. Especially suitable elastomers are nitrile rubber, ethylene-propylene-diene rubber, thermoplastic elastomers, fluoroelastomers or silicones, such as Elastosil^{®} or Silpurane.

In yet another embodiment, the seal is made from a hard thermoplastic material, which may include, for example, polytetrafluoroethylene (PTFE), polyurethane (PU), or fluorinated ethylene propylene (FEP). To form a seal from this material, a tubular piece of material is placed over the hollow needle and then heated and radially compressed so that part of the thermoplastic material melts and adheres to the surface of the hollow needle. After cooling, the seal formed in this way is in close frictional contact with the needle surface and thus prevents fluid from escaping between the seal and the hollow needle, but allows the hollow needle to be moved within the thermoplastic material without encountering any great resistance.

In an alternative embodiment, the seal consists of an elastomeric material that is coated at least in part with a layer of thermoplastic material, like PTFE. Coating with such a material reduces the friction between the seal and the hollow needle.

In another embodiment, the bushing, the spacer sleeve, and the seals are integrally formed from a thermoplastic material, like PTFE. Alternatively, only the bushing and the seal are integrally formed. Still alternatively, only the seals and the spacer sleeve are integrally formed.

The medical fluid dosing device with a cylinder and a piston which is mounted in the cylinder so as to be longitudinally displaceable and which forms with the cylinder a variable-volume working chamber, with an actuator which displaces the piston along the longitudinal extent of the cylinder, with a fluid reservoir which is fluidically coupled to the cylinder via a supply line, with a discharge line which leads from the cylinder to a discharge point, provides at least one valve which has a hollow needle with an inlet opening, a closed tip and a radial side opening, wherein the hollow needle is mounted so as to be longitudinally displaceable in a bushing between a closed position and an open position and is characterized in that, in the open position, the side opening and the closed tip project into a lumen from which an outlet channel leads.

In one embodiment, the medical fluid dosing device has a connecting element into which the cylinder, the supply line and the discharge line open. Due to the small dimensions of the cylinder and the supply line as well as the discharge line, these open into a common connecting element, as the cylinder and its wall are too small to form a stable receptacle for the supply line and the discharge line.

In one embodiment, the piston is designed as a wire, with a seal being arranged between the cylinder and the wire in order to seal the working chamber formed by the piston and the cylinder. Advantageously, the seal is attached to the cylinder and slides along the piston during the piston movement. The piston is therefore a plunger that is sealed off from the environment by a seal that is statically arranged in the cylinder. The result of such an arrangement is that a high pressure can be built up with comparatively low friction. With a seal arranged on the piston and moving relative to the cylinder, a frictional force is generated which acts against the seal, causing a reaction force which counteracts the displacement movement of the piston. The plunger design takes account of the need to generate the lowest possible frictional forces in order to achieve precise displacement with the lowest possible drive forces.

In one embodiment, at least one of the valves is designed as a control valve in order to effect a controlled, in particular a delayed release of the fluid from the outlet channel when a sufficiently high pressure has built up in the working chamber. This prevents the fluid from being unintentionally pumped back into the inlet, which would reduce the amount of fluid to be supplied to the patient. It also prevents an unintentional administration of fluid.

The control valve can be coupled to the actuator of the piston or to a separate drive. A coupling with the actuator of the piston reduces the size and weight of the fluid dosing device. A separate actuator can be used to achieve independent switching of the respective control valve.

In one embodiment, the control valves are coupled with a control circuit that only opens the first control valve in the inlet port when the piston has enlarged the working chamber, i.e. when it has moved from an upper end position with a minimum working chamber volume towards a lower reversal position. In particular, the second valve is only opened when the working chamber has been reduced, i.e. when a reversing movement of the piston has already taken place after a desired maximum volume of the working chamber has been reached, so that excess pressure has built up within the working chamber.

In one embodiment, the actuator has a shape memory alloy, an electric motor or a solenoid as a drive for the piston. A shape memory alloy is comparatively cheap and light and is therefore very suitable for an implanted fluid dosing device. In particular, if the piston has to be displaced against a preload force from an initial position to a working position, a shape memory alloy acting on one side is a preferred drive. The starting position is, for example, the lower piston position, in which the working chamber has a maximum volume. With a servomotor in the form of an electric motor, the piston can easily be driven in both directions so that the piston can be moved to any desired position with the driving force of the motor. Compared to a shape memory alloy, a solenoid valve, relay or solenoid is structurally more complex and has moving parts that can wear out. The imprecise positioning of the piston inherent in a shape memory alloy actuator is compensated for in the present invention by the defined volume of fluid delivery through the maximum stroke of the piston within the cylinder.

Independently of the drive, in one embodiment the piston is coupled to a spring which applies a preload force to the piston in the direction of a retracted position with a maximum working chamber volume, so that the working chamber is reduced when the spring is released.

All the elements and design examples described above can be combined with each other.

In the following, embodiments of the invention are explained in more detail with reference to the figures. Identical reference signs denote identical components and elements. For reasons of clarity, reference signs may have been omitted in some figures. The figures show
Figure 1 A- a valve in a partial sectional view in a first position;
Figure 1 B - the valve shown in Figure 1 A in a second position;
Figure 2 A - a variant of the valve not covered by the invention in a first position;
Figure 2 B - the valve shown in Figure 2 A in a second position;

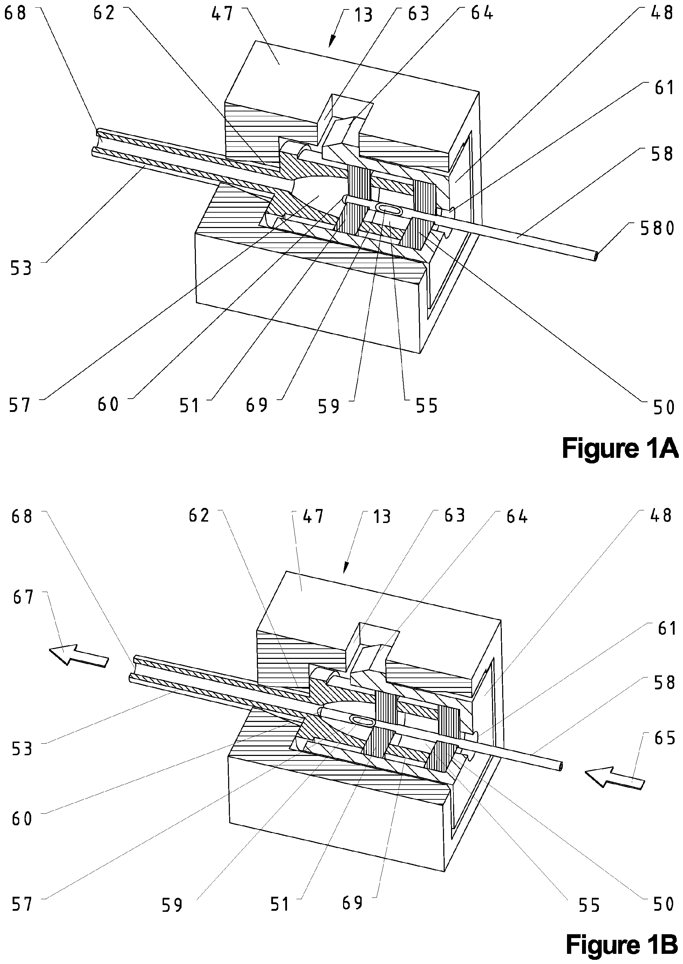

Figure 3 A - a variant of Figure 1 in a first position;

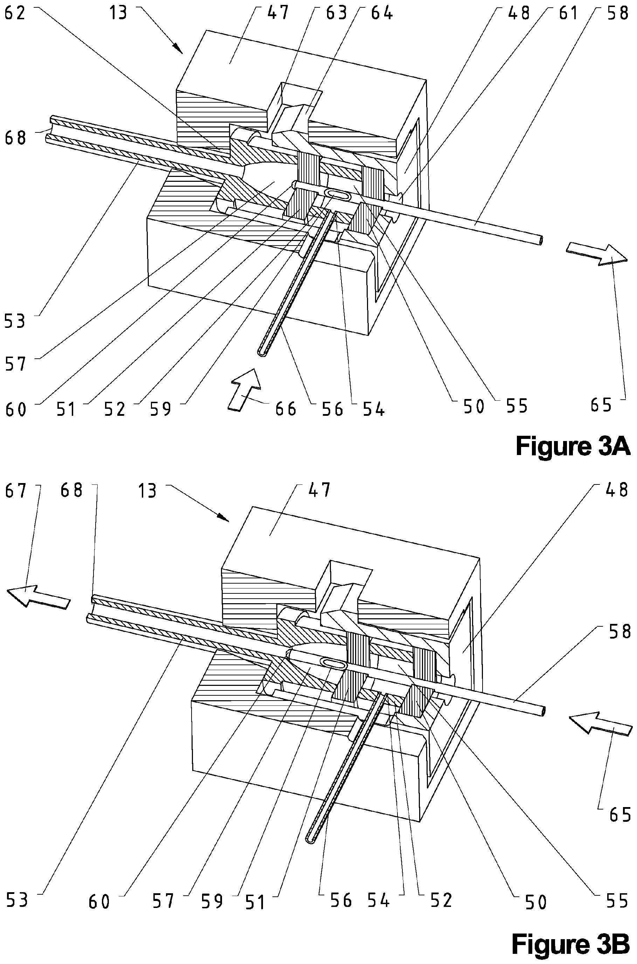

Figure 3 B - the valve shown in Figure 3 A in a second position;

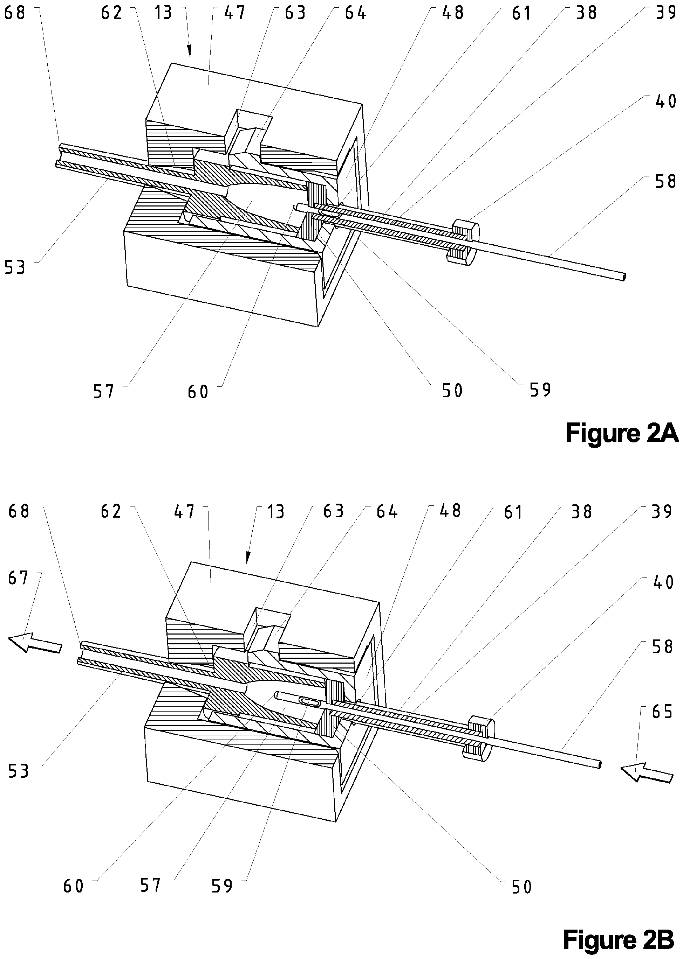

Figure 4 A - a variant of Figure 2 A;

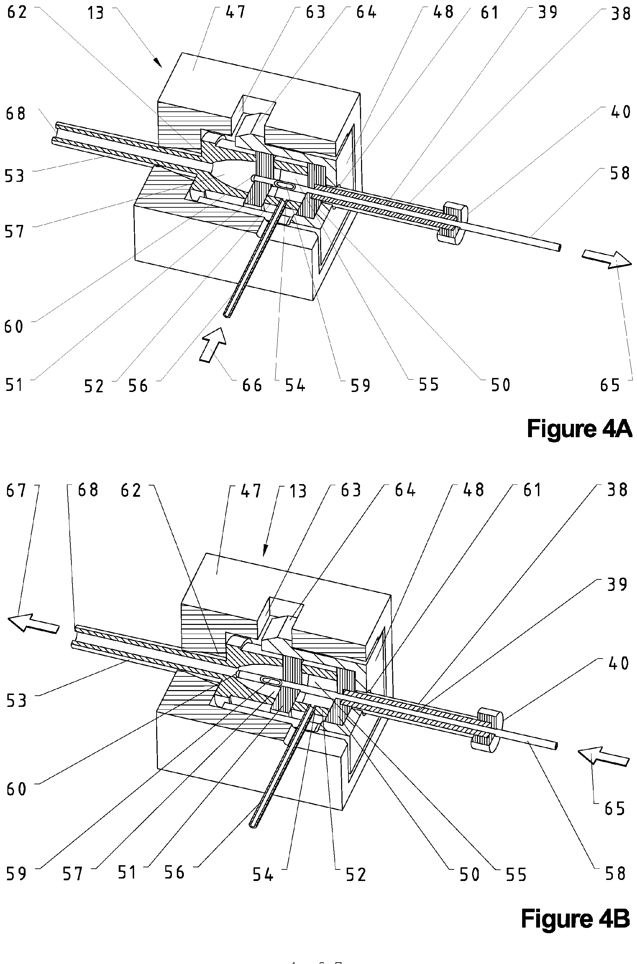

Figure 4 B - the valve shown in Figure 4 A in a second position;

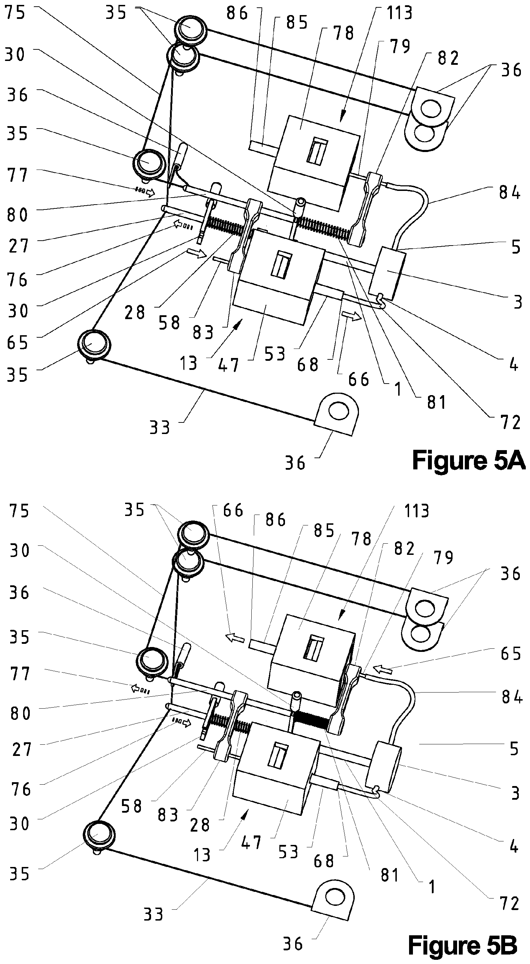

Figure 5 A - Illustration of a fluid dosing device in a first position;
Figure 5 B - the device shown in Figure 5 A in a second position;
Figure 6 A - a variant of the fluid dosing direction;

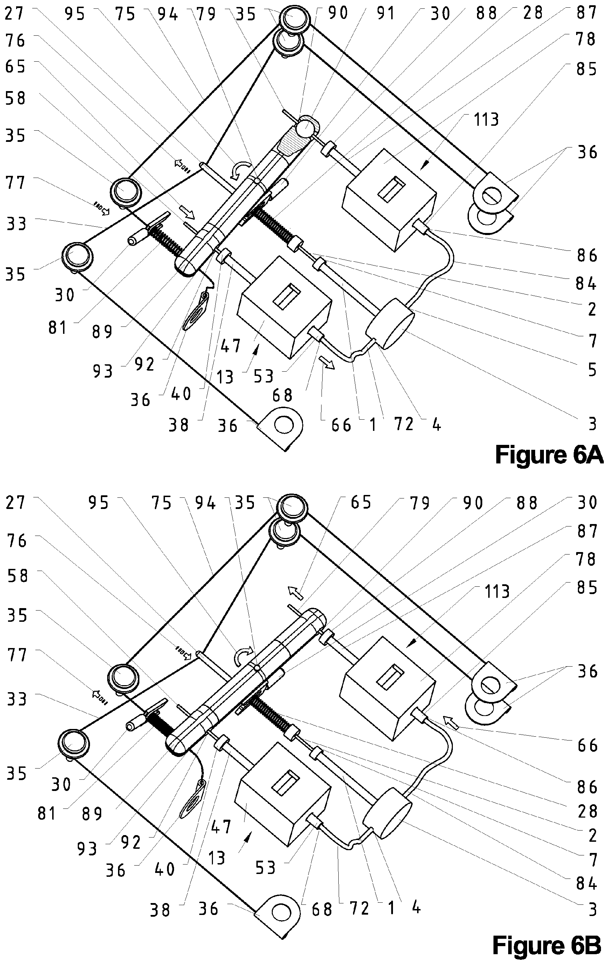

Figure 6 B - a device according to Figure 6 A in a second position;
Figure 7 A - a variant of the fluid dosing device; and

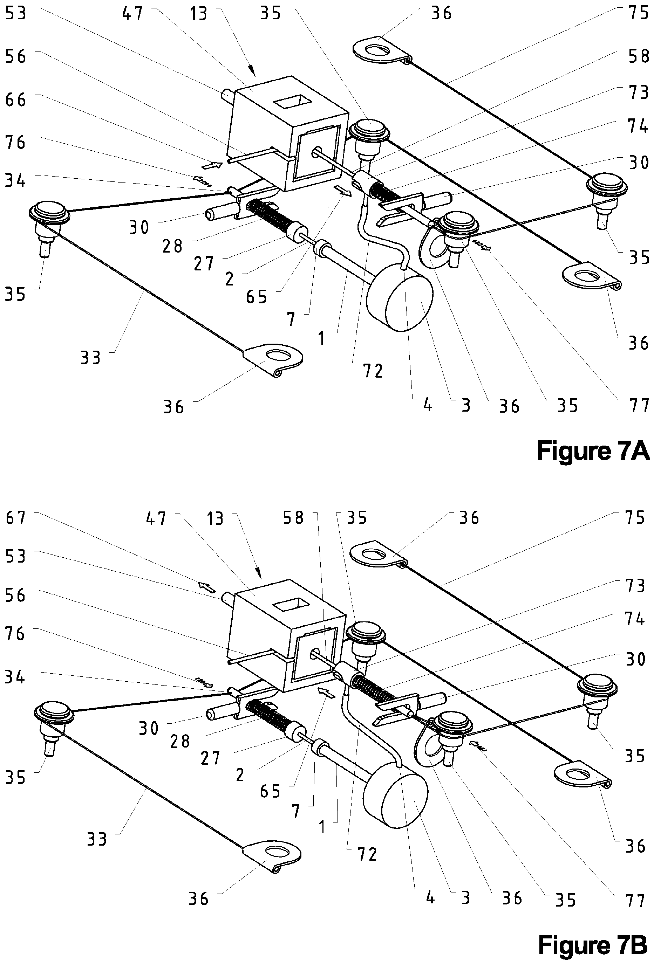

Figure 7 B - the device shown in Figure 7 A in a second position. I

Figure 1 A shows a first variant of a valve for a medical fluid dosing device, with a hollow needle 58 having an inlet opening 580, a closed tip 60 and a lateral opening 59 spaced apart from the closed tip 60. In the position of the hollow needle 58 shown, the lateral opening 59 is located between a first seal 51 and a second seal 50. The first seal 51 and the second seal 50 seal the hollow needle 58 to the outside and delimit a spacer sleeve 69, which keeps the first seal 51 and the second seal 50 spaced apart from each other. Within the space between the first seal 51 and the second seal 50 and the spacer sleeve 69, there is a free space 55 in which the fluid that is or is to be transported through the hollow needle 58 can enter. In the illustrated position of the hollow needle 58, the closed end 60, which can be produced by crimping, welding, filling, soldering or other types of sealing, projects into a lumen 57 arranged in a bushing 53 extending out of a valve housing 47. The bushing 53 ends with an opening 68 as the end of a channel within the bushing 53. The bushing 53 is inserted into an opening 62 of the valve housing 47 and ends at the second seal 51. The bushing 53 has a shoulder which abuts against a corresponding abutment surface of the valve housing 47 and is releasably secured to the valve housing 47. It is secured via the seal holder 48, which is inserted on the front side of the valve housing 47. The seal holder 48 has an opening 61 through which the hollow needle 58 is inserted.

The seal holder 48 is held positively in the valve housing 47 via a projection 64, which is directed radially outwards and engages in an opening 63 within the valve housing 47. In the illustrated embodiment example, the opening 63 is arranged on the upper side of the valve housing 47. In the illustrated embodiment example, the seal holder 48 is provided with an angular, in particular square cross-section and has a substantially cylindrical bore into which the bushing 53, the first and second seal 50, 51 and the spacer sleeve 69 are inserted. The opening 61 in the rear wall of the seal holder 48 is smaller than the outer diameter of the first seal 50, so that when the seal holder 48 is inserted into the valve housing 47, the components are pressed together axially and locked tightly in the valve housing.

In Figure 1 B, the valve arrangement according to Figure 1 A is shown in a second position, in which the hollow needle 58 has been pushed out of the spacer sleeve 69 between the first and second seal 50, 51 into the lumen 57 of the bushing 53 until the lateral opening 59 of the hollow needle protrudes into the lumen 57. If fluid is now passed through the hollow needle 58 in the direction of the arrow 65, the fluid flows through the hollow needle 58 and the lateral opening 58 into the lumen 57 and from there through the bushing 53 to the open end 68 and to the desired location, which is indicated by the arrow 67.

A variant of the valve structure not covered by the invention is shown in a partial sectional view in Figure 2 A and 2B. Here, a hollow needle 58 with a closed tip 60 and a lateral opening 59 is partly located in a valve housing 47, and in a capillary 38 that is fixed to the seal holder 48. The capillary 38 is inserted into the opening 61 within the seal holder 48 and ends in the first seal 50, which in turn is arranged at the rear end of the recess within the seal holder 48. By mounting the seal holder 48 in the valve housing 47, the first seal 50 is fixed in the holder 48 by the contact pressure exerted on the seal 50 by the bushing 53 A free space 39 is formed between the capillary 38 and the hollow needle 58, which corresponds functionally to the free space 55 according to the embodiments of Figures 1 A and 1 B. A seal 40 is arranged at the front end of the capillary 38 to seal the free space 39 from the environment. In the position of the hollow needle 58 shown, the valve is closed. If the hollow needle 58 is inserted into the lumen 57, as shown in Figure 2 B, the valve is opened. Fluid is introduced in the direction of the arrow 65 through the hollow needle 58 into the lumen 57 through the lateral opening 59. The fluid flows out of the lumen 57 through the bushing 53 to the outlet opening 68 in the direction of flow 67 and from there to a dispensing point at which the fluid is administered. The bushing 53 with the lumen 57 formed therein has a substantially constant diameter at the front end, the volume tapering from the first seal 50 initially slightly, then more strongly in the direction of the outlet channel towards the opening 68.

Figure 3A shows a structure of a valve which essentially corresponds to the structure of the valve according to Figure 1 A. Instead of a closed cavity 55 within the spacer sleeve 52, which is arranged between the first and second seal 50, 51, there is an inlet component 56 in the side wall of the spacer sleeve 52, which can be designed as a rigid tube, for example. The inlet component 56 penetrates laterally through the valve housing 47 and is arranged in an opening 54 of the spacer sleeve 52 and establishes a fluidic connection of the cavity 55 with a conveying device or a reservoir for fluid. Thus, in the position shown in Figure 3 A, which corresponds to the closed position of the valve according to Figure 1 A, fluid can be conveyed from a conveying device or a storage container through the inlet component 56 according to the arrow 66 into the cavity 55. From there, the fluid passes through the lateral opening 59 through the hollow needle 58 away from the closed end 60, for example to a pump arrangement whose delivery volume is being increased. This can be caused, for example, by a displacement of a piston, so that fluid in this position can be sucked in from a reservoir through the inlet component 56 and the hollow needle 58. Once the suction process is complete, the hollow needle 58 is then displaced in the direction of the lumen 57, so that the lateral opening 59 establishes a fluidic connection with the lumen 57. The second seal 51 is so thick that it covers the entire lateral opening 59 so that no fluid can flow between the inlet component 56 and the lumen 57 when the lateral opening 59 is moving through the second seal 51.

If the hollow needle 58 is in the position shown in Figure 3 B, there is a hydraulic separation between the cavity 55 and the lumen 57 and the fluid, which was initially drawn in through the hollow needle 58, is conveyed in the direction of the arrow 61 into the lumen 57 and from there through the outlet 68 to the discharge point. In this position, no fluid can pass from the inlet component 56 into the lumen 57 or into the hollow needle 58.

Figures 4 A and 4 B show the two positions of the arrangement shown in Figures 2 A and 2 B. Here too, analogous to the embodiment according to Figures 3 A and 3 B, an inlet component 56 is arranged, which projects into the valve housing 47. In deviation from the embodiments of Figures 2 A and 2 B, the lumen 57 is subdivided by a second seal 51 or the cavity 55 is formed by a spacer sleeve 52 analogous to the embodiment according to Figure 1 A. Figure 4 A is thus a functional combination of Figures 2 A and 3 A. In Figure 4 B, the hollow needle 58 is displaced beyond the second seal 51 in the direction of the outlet 68, so that fluid is only conveyed through the hollow needle 58 into the lumen 57 and from there through the bushing 53 to the discharge point in the direction of the arrow 67. In the embodiment shown in Figures 4 A and 4 B, there is also a hydraulic separation between the inlet component 56 and the outlet opening 68 of the bushing 53. The second seal 51 is dimensioned and designed accordingly. The inlet 54 in the spacer sleeve 52 is sealed with respect to the inlet component 56 and is formed, for example, as a bore with a seal or a conical design. Corresponding lateral inlet openings 54 are also present in the embodiment according to Figures 3A and 3 B.

Figure 5 A shows a schematic representation of a fluid dosing device with two valves, as shown, for example, in Figures 1 A, 1 B or Figures 2 A, 2 B. The valves 13, 113 are arranged in a first valve housing 47 and a second valve housing 78, respectively. Fluid is guided in the direction of flow according to the arrow 65 through the first hollow needle 58 into the first valve housing 47 and from the lumen 57, which is not shown, through the bushing 53 to the outlet opening 68 of the first valve. There, the fluid is guided in the direction of the arrow 66 through a line 72, for example a flexible line, to an inlet 4 of a connecting element 3, which is coupled to a capillary 1. A plunger, not shown, is displaceably arranged in the capillary 1 and is driven via a drive, e.g. a shape memory element 53, here in the form of a wire, which is coupled to wire crimps 36 via deflection rollers 35. This takes place, for example, by heating and cooling. The direction of movement is indicated by the arrow 76. A spring seat 27, to which the plunger, not shown, is attached, is pulled out, causing the preload spring 28, which is supported against a spring plate 30 under preload of the spring 28, to be displaced. This increases the volume within the capillary 1 and fluid is drawn in through the first valve 13 when the valve is in the open position. When the plunger is displaced back towards the connecting element 3 due to the preloading spring 28, fluid is pumped through the outlet 5 and the line 84 to the second hollow needle 79 of the second valve 113 with the second valve housing 78. In the open position of the valve, fluid is then conveyed through the second bushing 85 to the outlet 86.

The positions of the hollow needles 58, 79 are controlled via movably mounted crossbars 82, 83. The crossbars 82, 83 are each rigidly connected to the hollow needles 79, 58 and are longitudinally displaceably mounted along the longitudinal extension of the hollow needles 58, 79 on a spring seat 80. The spring seat 80 is arranged substantially parallel to the longitudinal extent of the hollow needles 58, 79 and can be displaced longitudinally by a second shape memory element 75. The second spring seat 80 is supported against a second preload spring 81, which is supported against one of the second spring plates 30. If the second shape memory element 75 is activated, the second spring seat 80 moves along the longitudinal extension and tensions the second preload spring 81. If the preload spring 81 relaxes, the second spring seat 80 is displaced in the direction of arrow 77, as shown in Figure 5. This moves the second hollow needle 79 into the closed position and simultaneously moves the first hollow needle 58 into the open position. This opens the first valve 13. Driven by a first spring seat 27 moving to the left in the direction of arrow 76, fluid flows, as shown by the arrows, through the first valve 13 and the connecting element 3 into the capillary 1.

If, as shown in Figure 5 B, the direction of movement is then reversed, i.e. the shape memory element 33 is relaxed and the second shape memory element 75 is tensioned, the hollow needles 58, 79 move in opposite directions. The first hollow needle 58 is displaced to the left along the direction of arrow 77 and brought into the closed position, while at the same time the second preload spring 81 is tensioned and the second hollow needle 79 is brought into the open position. When the first shape memory element 33 is released, the first preload spring 28 is released and the plunger is moved along the capillary 1 in the direction of the coupling element 3 and pushes the initially aspirated fluid through the line 84 through the second valve 113 to the outlet 86.

A second embodiment of the fluid dosing device is shown in Figures 6 A and 6B in different positions. Here, too, the valves 13, 113 are arranged with two valve housings 47, 78 and coupled with a connecting element 3. The capillary 1 with the plunger 2, a preload spring 28 and the spring seat 27 are present as described in Figures 5 A and 5 B and are moved in one direction or the other by corresponding actuators, for example the shape memory element 33. The valves 13, 113 are designed according to the embodiments of Figures 2 A, 2 B and have a hollow needle 58, 79, which is located in a capillary 38, 87 and is sealed off from the environment by a seal 40, 88. In contrast to Figures 2 A and 2 B, the fluid is not introduced from the connecting element 3 into the second valve 113 via the hollow needle 79, but via the opening of the second bushing 85, so that the flow direction is reversed and the fluid is introduced into the lumen through the channel in the bushing 85. When the first valve 13 is in the open position, the fluid is transported through the lateral opening 59, not shown, and through the hollow needle to the desired location. The hollow needle 58, 79 is mounted on a coupling rod 89, which is pivotably mounted about a pivot axis 94. The pivot axis 94 is advantageously perpendicular to a plane in which the two hollow needles 58, 79 are oriented. Suitable bearings can be used to compensate for small deviations.

On the second valve 113, the coupling rod 89 is coupled to the second hollow needle 79 via a ball joint 91. The ball joint 91 is mounted in a first bore 90 in the coupling rod 89; a corresponding mounting takes place on the first hollow needle 58. The respective hollow needle 79, 58 passes through the bores 90, 92. The coupling rod 89 is actuated via a shaped memory element which is attached to the wire crimp 36. The coupling rod 89 is preloaded accordingly via a preload spring 81, which is supported on a spring plate 30, so that the second shape memory element 75, which passes through the preload spring 81, displaces the coupling rod 89 in one direction or the other about the pivot axis 94 by tension or relaxation. In the position shown in Figure 6 A, the first shape memory element 33 is initially tensioned. As a result, the first preload spring 28 is also tensioned and the plunger 2 is pulled out of the capillary 1. This creates a vacuum in the capillary 1 and the connecting element 3. Simultaneously the second shape memory element 75 is relaxed. This opens the first valve 13, as the coupling rod 89 pivots about the pivot axis 94 in the direction of arrow 95. At the same time, the second hollow needle 79 is moved in the opposite direction and brought into the closed position. Fluid can therefore flow, as shown by the arrows 65, 66, through the first valve 13 and the connecting element 3 into the capillary 1.

In order to then pump fluid out of the capillary 1, the second shape memory element 75 is tensioned so that the second preload spring 81 is tensioned and the coupling rod 89 is displaced in the opposite direction. As a result, the hollow needle 58 of the first valve 13 is moved into the closed state, so that no further fluid can be drawn in or forced through the first valve 13. Together with the movement of the first hollow needle 58, the second hollow needle 79 is displaced into the open position, thereby bringing the second valve 113 into the open state. The first shape memory element 33 is released, which simultaneously releases the first preload spring 28 and presses the plunger 2into the capillary 1, causing fluid to flow from the capillary 1 through the connecting element 3 into the line 84. Fluid previously sucked in via the first valve 13 cannot be transported back but now is conveyed through the second valve 113 to the discharge point as shown by arrows 65 and 66.

Figures 7 A and 7 B show a further variant of the fluid dosing device. Instead of two valves that can be switched between two states, the embodiment of Figures 7 A and 7 B uses a valve 13 that has an additional inlet component 56. Such functionality is explained in connection with Figures 3 A and 3B as well as 4 A, 4 B. Actuation of the plunger 2 with the capillary 1 takes place via the first shape memory element 33, as described above. When the first shape memory element 33 is tensioned, the spring seat 27 is pulled in the direction of the arrow 76 against the preload force of the spring 28. As a result, the plunger 2, which is coupled to the spring seat 27, is pulled out of the capillary 2 and fluid is conveyed out of the first valve 13 through the line 72. The hollow needle 58 is in the position shown in Figures 3A and 4 A, in which fluid can now be drawn in through the hollow needle 58 via the inlet component 56. The hollow needle 58 is biased by a second biasing spring 74, which is supported in a spring seat 73 and with respect to a spring plate 30. The hollow needle 58 is actuated via the second shape memory element 75, which is coupled to the second spring seat 73 via deflection rollers 35.

In order to move the aspired fluid out of the capillary 1, the first shape memory element 33 is released, causing the preload spring 28 to relax and the plunger 2 to push the fluid out of the capillary 1 in the direction of the hollow needle 58. The fluid then flows out of the outlet 4 through the line 72 into the hollow needle 58. The hollow needle 58 has been previously moved to the position shown in Figures 3 B, 4 B, in which the lateral opening 59 is located within the lumen 57 and a fluidic connection is thereby established between the capillary 1, the outlet 4, the line 72, the hollow needle 58 and the outlet 68 of the bushing 53, so that fluid can be discharged in the direction of the arrow 67.

## Claims

1. Valve for a medical fluid dosing device with a cylinder (1) and a piston (2), which is mounted in the cylinder (1) so as to be longitudinally displaceable and forming a variable-volume working chamber (6) with the cylinder (1), with an actuator (33), which displaces the piston (2) along the longitudinal extension of the cylinder (1) and draws the fluid from a fluid reservoir (9), which is fluidically coupled to the cylinder (1), and pumps it through the valve (13) to a discharge point (23) when the movement is reversed, wherein the valve (13) has a hollow needle (58) with an inlet opening (580) for the fluid, a closed tip (60) opposite the inlet opening (580) and a radial side opening (59), the hollow needle (58) is mounted longitudinally displaceably in a bushing (53) between a closed position and an open position, wherein in the open position the side opening (59) and the closed tip (60) project into a lumen or chamber (57), from which an outlet channel (68) leads, **characterised in that** in the closed position the side opening (59) is arranged between two seals (50, 51) in a spacer sleeve (69).

2. Valve according to claim 1, **characterised in that** the seal (50, 51) is arranged in a seal holder (48) which is fixed to the bushing (53).

3. Valve according to one of the preceding claims, **characterised in that** the hollow needle (58) is guided through a capillary (38) into the bushing (53).

4. Valve according to claim 3, **characterised in that** the capillary (38) has a seal (40) on the end face which surrounds the hollow needle (58).

5. Valve according to one of the preceding claims, **characterised in that** the bushing (53) is inserted in a valve housing (47, 78) and is positively secured.

6. Valve according to one of the preceding claims, **characterised in that** the bushing (53) and the outlet channel (68) are formed in one piece.

7. Valve according to one of the preceding claims, **characterised in that** the valve (13, 113) is coupled to an actuator (33, 75).

8. Valve according to one of the preceding claims, **characterised in that** an inlet line (56) opens into the spacer sleeve (69) and is in fluidic connection with the side opening (59) in the closed position.

9. Valve according to one of the preceding claims, **characterised in that** the seal (50, 51) is not wider than the length of the side opening (59).

10. Valve according to claim 7, **characterised in that** the actuator (33, 75) is designed as a spring or shape memory alloy.

11. Medical fluid dosing device with a valve (13) according to one of the preceding claims with a cylinder (1) and a piston (2), which is mounted in the cylinder (1) so as to be longitudinally displaceable and forms a variable-volume working chamber (6) with the cylinder (1), with an actuator (33) which displaces the piston (2) along the longitudinal extent of the cylinder (1) and draws the fluid from a fluid reservoir (9) which is fluidically coupled to the cylinder (1) and, when the movement is reversed, pumps it through the valve (13, 113) and the outlet channel (68) to a discharge point (23).

12. Medical fluid dosing device according to claim 11, **characterised in that** the valve (13, 113) is coupled to the actuator (33) of the piston (2) or to a separate drive (75).
